# EUROPÄISCHE PATENTSCHRIFT

(11) **EP 1 126 800 B1**
(45) Veröffentlichungstag und Bekanntmachung des Hinweises auf die Patenterteilung: **31.03.2004**
(21) Anmeldenummer: 99955956.0
(22) Anmeldetag: 04.11.1999
(51) Int. Cl.: A61F 5/455

(54) **WEIBLICHES URINAL**
FEMALE URINAL
URINAL FEMININ

(30) Priorität: 06.11.1998 DE 19851267
(43) Veröffentlichungstag der Anmeldung: 29.08.2001
(73) Patentinhaber: Mutke, Hans Guido, 81477 München (DE)
(72) Erfinder: Mutke, Hans Guido, 81477 München (DE)
(74) Vertreter: Vogeser, Werner, Dipl.-Ing.
(86) Internationale Anmeldenummer: PCT/EP1999/008450
(87) Internationale Veröffentlichungsnummer: WO 2000/027320

(56) Entgegenhaltungen:
- FR-A- 2 590 160
- US-A- 3 194 238
- US-A- 3 601 125
- US-A- 4 815 151
- US-A- 5 411 495

## Beschreibung

Die Erfindung betrifft ein Femurinal.

Aus der US 3 194 238 ist ein Femurinal bekannt, das so ausgestaltet ist, daß bei unwillkürlichem Abgang von Urin dieser in einen an einem Bein der Benutzerin befestigten Behälter geleitet wird.

Bei Inkontinenzproblemen, die zu einem unwillkürlichen Abgang von Urin führen können und die bei Frauen bereits nach eine Geburten und im fortgeschrittenen Alter auftreten können, ist es bekannt, saugfähige Einlagen zu verwenden, deren Kapazität begrenzt ist.

Dokument US-A-3 601 125 offenbart ein Femurinal, bestehend aus einem im wesentlichen gewölbten, flexiblem Urinableit- und Sammelkörper, der bei Gebrauch an den sich zwischen den Beinen einer Benutzerin anlegbar ist, am Umfang einem Dichtwulst aufweist, in Gebrauchslage im untersten Bereich mit einem flexiblen Schlauch zum ableiten von Urin versehen ist, wobei der Urinableit Körper mittels eines hinteren, bei Gebrauch vom Damm durch die Gesäßbacken einer Benutzering geführten Bandes an einem oberhalb der Beckenkammes einer Benutzerin tragbaren Gürtel fixierbar ist.

Der Erfindung liegt die Aufgabe zugrunde, ein Femurinal zu schaffen, das es ermöglicht, jederzeit und insbesondere im Stehen Urin lassen zu können.

Gelöst wird diese Aufgabe gem. der Erfindung durch die im Anspruch 1 angegebenen Merkmale. Zweckmäßige Ausgestaltungen der Erfindung ergeben sich aus den Unteransprüchen.

Das Femurinal dient bei heruntergeklapptem Schlauch zum direkten, spontanen Urin lassen, kann aber auch kurzzeitig als Reservoir verwendet werden.

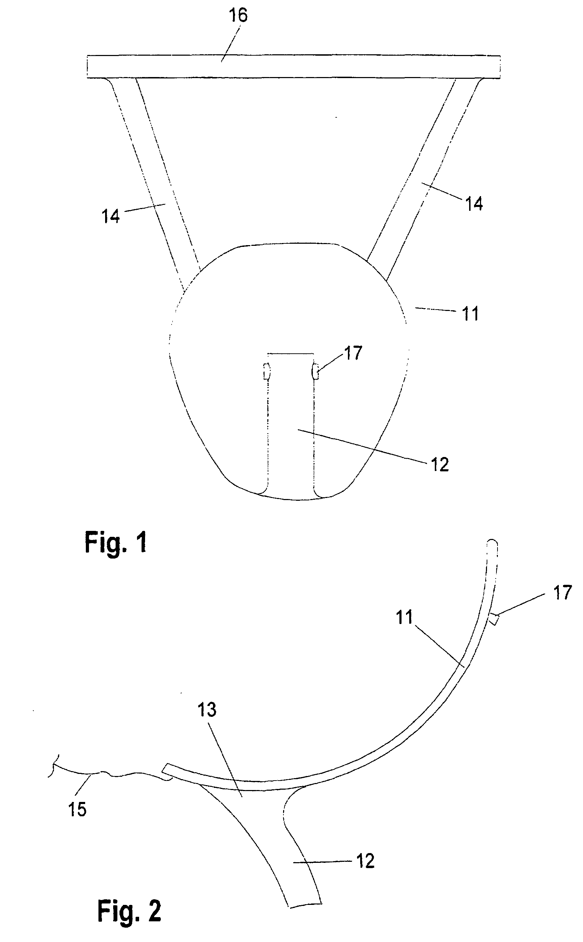

Die Erfindung wird nachstehend anhand der Fig. 1 und 2 beispielsweise beschrieben. Es zeigt:
Fig. 1 eine Aufsicht des Femurinals, und
Fig. 2 eine Seitenansicht davon.

Das Femurinal besteht aus einem gewölbten, flexiblen Urinsammel- und Urinableitkörper 11, der den Bereich zwischen den Beinen einer Benutzerin von etwa oberhalb des Schamhaares bis zum Damm zwischen After und hinterer Scheidenöffnung abdeckt und im unteren Bereich Urin auffangen kann.

Am Umfang ist der Ableitkörper 11 mit einem Dichtwulst (nicht gezeigt) versehen, der im Bereich des hinteren Scheideneingangs und des Afters einer Benutzerin aus weichem Material besteht. Der Ableitkörper 11 hat im untersten Bereich, in dem sich Urin ansammelt, eine trichterförmige Öffnung 13, die in einen flexiblen Schlauch 12 mündet. Das freie Ende des Schlauchs 12 kann unter Abbiegen etwa senkrecht an der Außenseite des Ableitkörpers 11 mittels einer schematisch gezeigten Druckoder einer Klettverschlußfixierung 17 abdichtend festgelegt werden. Zum Ableiten des Urins wird der Schlauch aus der Fixiereinrichtung gelöst und in die in Fig. 2 gezeigte Position gebracht.

Der Schlauch 12 hat vorzugsweise eine Länge von 15 -20 cm, so daß es möglich ist, ohne Ausziehen eines Slips durch Herunterklappen des Schlauchs 12 Urin abzulassen.

Am Schlauchende kann ein Gewinde vorgesehen sein, um ggf. über ein Zwischenschlauchstück einen an einer der Oberschenkelinnenseiten befestigbaren Behälter angeschlossen zu werden. Der Querschnitt des Schlauchs 12 kann zweckmäßigerweise oval sein, so daß er nicht aufträgt und wenig Platz beansprucht.

Die Befestigung des Femurinals erfolgt mittels zweier Bänder 14, die oben seitlich am Ableitkörper 11 befestigt sind, und beim Gebrauch in Richtung der Leisten einer Benutzerin verlaufen, und mittels eines unteren, hinteren, in Fig. 2 schematisch gezeigten Bandes 15, das vom Damm durch die Gesäßbacken einer Benutzerin geführt und am Gürtel 16 oberhalb des Beckenkamms fixierbar ist. Das untere, hintere Band 15 ist etwas unterhalb des Umfangsdichtwulstes befestigt, so daß der Wulst beim Gebrauch zur besseren Abdichtung einen Druck auf den Damm zwischen dem hinteren Scheideneingang und After einer Benutzerin ausübt.

Der Ableitkörper 11 kann im Bereich der in den Schlauch mündenden Öffnung 13 zur Erhöhung der Flexibilität dünner als im restlichen Teil des Ableitkörpers 11 ausgebildet sein.

Das Femurinal kann auch fest in einen Slip eingearbeitet sein, so daß er zusammen mit diesem gewaschen werden kann. Im oberen Teil kann der Ableitkörper 11 auch mit einem Deodorantpäckchen versehen werden.

Das Femurinal kann selbstverständlich in verschiedenen Größen und Formen je nach Bedarf angefertigt werden.

## Patentansprüche

1. Femurinal, bestehend aus einem gewölbtem, flexiblem Urinableit- und Sammelkörper (11), der bei Gebrauch an den sich zwischen den Beinen von etwa oberhalb des Schamhaares bis zum Damm erstreckenden Bereich einer Benutzerin anlegbar ist, am Umfang einen Dichtwulst aufweist, in Gebrauchslage im untersten Bereich mit einem flexiblen Schlauch (12) zum Ableiten von Urin versehen ist, wobei das freie Ende des Schlauches (12) unter Abbiegen des Schlauches (12) an der Außenseite des Urinableitkörpers (11) lösbar festlegbar ist und wobei der Urinableitkörper (11) mittels zweier, vorderer, bei Gebrauch in Richtung der Leisten einer Benutzerin verlaufender Bänder (14) und eines hinteren, bei Gebrauch vom Damm durch die Gesäßbacken einer Benutzerin geführten Bandes (15) an einem oberhalb des Beckenkammes einer Benutzerin tragbaren Gürtel (16) fixierbar ist.

2. Femurinal nach Anspruch 1,
**dadurch gekennzeichnet, daß**
der Dichtwulst im Bereich des hinteren Scheideneingangs und des Afters einer Benutzerin aus weichem Material besteht.

3. Femurinal nach Anspruch 1 oder 2,
**dadurch gekennzeichnet, daß**
das untere, hintere Band (15) etwas unterhalb des Dichtwulstes befestigt ist, um beim Gebrauch Druck auf den Damm zwischen dem hinteren Scheideneingang und dem After einer Benutzerin auszuüben.

4. Femurinal nach einem der Ansprüche 1 bis 3,
**dadurch gekennzeichnet, daß**
die in den Schlauch (12) mündende Öffnung (13) des Ableitkörpers (11) trichterförmig ausgebildet ist.

5. Femurinal nach Anspruch 4,
**dadurch gekennzeichnet, daß**
das Material des Ableitkörpers (11) wenigstens im Bereich der Öffnung (13) dünner als im restlichen Bereich des Sammelkörpers (11) ausgebildet ist.

6. Femurinal nach einem der Ansprüche 1 - 5,
**dadurch gekennzeichnet, daß**
der Schlauch (12) eine Länge von 15 - 20 cm hat.

7. Femurinal nach einem der Ansprüche 1 - 5,
**dadurch gekennzeichnet, daß**
der Querschnitt des Schlauchs (12) oval ist.

8. Femurinal nach einem der Ansprüche 1 - 6,
**dadurch gekennzeichnet, daß**
der Schlauch (12) am freien Ende mit einem Anschlußgewinde versehen ist.

## Claims

1. Female urinal consisting of a curved flexible urine draining and collecting body (11) which, when in use, can be placed against the region between a user's legs extending from approximately above the pubic hair to the perineum, has a sealing elevation on the circumference, is provided, in the usage position, with a flexible tube (12) in the lowest area, for draining urine, the free end of the hose (12), while the hose (12) is bent, being detachably fastenable on the exterior side of the urine draining body (11), and the urine draining body (11) being fixable on belt (16), which can be worn by a user above the iliac crest, by means of two frontal straps (14) which, when in use, extend in the direction of the user's groin and one rear strap (15) which, when in use, is guided from the perineum through the user's buttocks.

2. Female urinal according to Claim 1,
**characterized in that**, in the region of a user's rear vaginal entrance and anus, the sealing elevation consists of a soft material.

3. Female urinal according to Claim 1 or 2,
**characterized in that** the lower rear strap (15) is fastened slightly below the sealing elevation in order to exercise, when in use, pressure to the perineum between a user's rear vaginal entrance and anus.

4. Female urinal according to one of Claims 1 to 3,
**characterized in that** the opening (13) of the draining body (11) which. leads into the hose (12) is constructed in a funnel shape.

5. Female urinal according to Claim 4,
**characterized in that**, at least in the area of the opening (13), the material of the draining body (11) has a thinner construction than in the remaining area of the collecting body (11).

6. Female urinal according to one of Claims 1 to 5,
**characterized in that** the hose (12) has a length of from 15 to 20 cm.

7. Female urinal according to one of Claims 1 to 5,
**characterized in that** the cross-section of the hose (12) is oval.

8. Female urinal according to one of Claims 1 to 6,
**characterized in that** the hose (12) is provided with a connection thread at the free end.

## Revendications

1. Urinal féminin, constitué d'un corps flexible, bombé d'évacuation et de collecte de l'urine (11), qui peut être positionné à l'usage dans la zone s'étendant entre les jambes à partir environ de la moitié supérieure des poils pubiens jusqu'au périnée de l'utilisatrice, comporte à sa circonférence un bourrelet d'étanchéité, est muni en position d'utilisation d'un tube flexible (12) situé dans sa partie inférieure pour l'évacuation de l'urine, sachant que l'extrémité libre du tube (12) peut être fixée de façon amovible sous torsion du tube (12) vers le bord externe du corps d'évacuation de l'urine (11) et sachant que le corps d'évacuation de l'urine (11) peut être fixé à une ceinture (16) portable au-dessus de la crête iliaque de l'utilisatrice au moyen de deux bandes (14) avant s'étendant à l'usage en direction de l'aine de l'utilisatrice et d'une bande (15) arrière introduite à l'usage par le périnée au travers des fesses de l'utilisatrice.

2. Urinal féminin selon la revendication 1, **caractérisé en ce que** le bourrelet d'étanchéité dans la zone d'entrée arrière du vagin et de l'anus est constitué d'un matériau mou.

3. Urinal féminin selon l'une quelconque des revendications 1 ou 2, **caractérisé en ce que** la bande (15) arrière est fixée un peu en dessous du bourrelet d'étanchéité, pour exercer au cours de l'utilisation une pression sur le périnée entre l'entrée arrière du vagin et l'anus d'une utilisatrice.

4. Urinal féminin selon l'une quelconque des revendications 1 à 3, **caractérisé en ce que** l'ouverture (13) débouchant dans le tube (12) du corps d'évacuation (11) a la forme d'un entonnoir.

5. Urinal féminin selon la revendication 4, **caractérisé en ce que** le matériau du corps d'évacuation (11) au moins dans la zone de l'ouverture (13) est plus fin que dans le reste du corps de collecte (11).

6. Urinal féminin selon l'une quelconque des revendications 1 à 5, **caractérisé en ce que** le tube (12) a une longueur de 15-20 cm.

7. Urinal féminin selon l'une quelconque des revendications 1 à 6, **caractérisé en ce que** la coupe transversale du tube (12) est ovale.

8. Urinal féminin selon l'une quelconque des revendications 1 à 6, **caractérisé en ce que** le tube (12) est muni d'un filet de fixation à son extrémité libre.
